# EUROPÄISCHE PATENTSCHRIFT

(11) **EP 3 119 436 B1**
(45) Veröffentlichungstag und Bekanntmachung des Hinweises auf die Patenterteilung: **28.11.2018**
(21) Anmeldenummer: 15707957.5
(22) Anmeldetag: 05.03.2015
(51) Int. Cl.: A61K 47/32, A61K 47/34, A61K 47/36, A61K 9/70, A61K 8/02

(54) **FLÄCHENGEBILDE ZUR KONTROLLIERTEN FREISETZUNG VON WIRKSTOFFEN**
SHEET-LIKE STRUCTURE FOR THE CONTROLLED RELEASE OF ACTIVE SUBSTANCES
STRUCTURE PLANE POUR LA LIBÉRATION CONTRÔLÉE DE SUBSTANCES ACTIVES

(30) Priorität: 18.03.2014 DE 102014003731
(43) Veröffentlichungstag der Anmeldung: 25.01.2017
(73) Patentinhaber: Carl Freudenberg KG, 69469 Weinheim (DE)
(72) Erfinder: SCHÖPPING, Gerhard, 69469 Weinheim (DE); GEISS, Franka, 69509 Mörlenbach (DE); LANGE, Birger, 64295 Darmstadt (DE); BEYER, Daniela, 76327 Pfinztal (DE)
(86) Internationale Anmeldenummer: PCT/EP2015/054606
(87) Internationale Veröffentlichungsnummer: WO 2015/139965

(56) Entgegenhaltungen:
- EP-A1- 2 199 447
- DE-T2- 69 008 456
- DE-T2- 69 612 345
- US-A1- 2003 113 463
- US-A1- 2006 121 099

## Beschreibung

Die Erfindung betrifft ein Flächengebilde zur Freisetzung von Wirkstoffen.

### Stand der Technik

Flächengebilde zur Freisetzung von Wirkstoffen sind aus dem Stand der Technik bereits bekannt.

In der US 2006/0121099 A1 wird ein Verfahren zum Aufbringen eines Wirkstoffs auf die Oberfläche eines Vliesstoffes oder einer Cellulosematte gezeigt. Der mindestens eine Wirkstoff kann durch Flüssigkeit, Druck oder Reibung freigesetzt werden.

Die FR 2 858 637 A1 beschreibt ein textiles Vlies, das Mikrokapseln aufweist. Die Mikrokapseln enthalten eine aktive Substanz. Die aktive Substanz ist ein Parfüm, ein Deodorant, eine Abschminklotion, ein Sonnenschutzmittel, ein Schutzmittel für nach dem Sonnenbaden oder ein hygienisches Reinigungsprodukt für Kleinkinder.

Die DE 10 2013 003 755 zeigt einen Belüftungseinsatz zur Anordnung in oder auf Textilien. Der Belüftungseinsatz weist mindestens eine Lage auf, wobei die Lage ein Absorptionsmittel und Lüftungsöffnungen aufweist. Unter Einwirkung von Flüssigkeiten können aufgrund einer Quellung des Absorptionsmittels die Lüftungsöffnungen zumindest teilweise verschlossen werden.

Aus der US 2012/0209231 A1 sind Superabsorber enthaltende Textilien bekannt. Diese enthalten hydrophile Fasern, die nach der Aufbringung des Superabsorbers eingebracht werden. Durch die zusätzlichen hydrophilen Fasern wird die Flüssigkeit im Textil verteilt.

### Darstellung der Erfindung

Aufgabe der vorliegenden Erfindung ist es ein Flächengebilde zur Freisetzung von Wirkstoffen bereitzustellen, das verglichen mit bekannten Flächengebilden eine verbesserte Freisetzung von Wirkstoffen und deren verbesserte Aufnahme in die Haut ermöglicht. Ferner soll das Flächengebilde in der Lage sein Feuchtigkeit, insbesondere Wasser, zu absorbieren und hautpflegende Wirkstoffe abzugeben. Weiterhin soll das Flächengebilde in bestimmten Ausführungsformen die Haut pflegen, ein gutes Haftvermögen besitzen, schmerzfrei und rückstandslos entfernt werden können und ein angenehmes Tragegefühl auf der Haut erzeugen.

Die vorliegende Erfindung löst die zuvor genannte Aufgabe durch die Merkmale des Patentanspruchs 1.

Danach zeichnet sich das eingangs genannte Flächengebilde dadurch aus, dass es einen mit mindestens einem Wirkstoff versehenen Grundkörper aus einem textilen Material umfasst, wobei der Grundkörper Fasern und einen Superabsorber als feuchtigkeitsregulierendes Mittel aufweist und der mindestens eine Wirkstoff in einer Polymermatrix eingebettet ist, wobei die Fasern zumindest teilweise stoffschlüssig mit dem feuchtigkeitsregulierendem Mittel verbunden sind.

Erfindungsgemäß ist erkannt worden, dass das Flächengebilde aufgrund einer synergistischen Kombination aus einem in einer Matrix eingebetteten Wirkstoff und einem feuchtigkeitsregulierenden Mittel eine verbessere Freisetzung des Wirkstoffs und eine verbesserte Aufnahme des Wirkstoffs in die Haut ermöglicht. So kann zwischen Flächengebilde und Haut aufgrund von im feuchtigkeitsregulierenden Mittel gespeicherter Flüssigkeit eine feuchte Atmosphäre erzeugt werden wodurch die Aufnahme und/oder Wirkung des Wirkstoffs unterstützt und verbessert werden kann.

Ferner kann durch die im feuchtigkeitsregulierenden Mittel gespeicherte Flüssigkeit ein optimales Tragegefühl auf der Haut erzeugt werden.

Ein weiterer Vorteil ist, dass insbesondere bei einem engen Kontakt zwischen Grundkörper und Haut ein intensiver Okklusionseffekt erhalten werden kann. Unter Okklusionseffekt wird ein Feuchtigkeitsstau in den oberen Hautschichten, bevorzugt in der Hornschicht der Haut (Stratum corneum), verstanden, wodurch eine Quellung der Haut erfolgen kann. Durch das erhöhte Flüssigkeitsangebot kann das Eindringen von kosmetischen und pharmazeutischen Stoffen erhöht werden, wodurch der Feuchtigkeitsgehalt im Flächengebilde und in der damit bedeckten Haut konstant gehalten und die Wirkung des Wirkstoffs maximiert werden kann.

Ein besonders intensiver Okklusionseffekt kann durch die Verwendung eines quellbaren feuchtigkeitsregulierenden Mittels erzielt werden.

Des weiteren kann die im feuchtigkeitsregulierenden Mittel gespeicherte Flüssigkeit auch als Diffusionsmedium für den Wirkstoff dienen. Ein kleiner Teil der gespeicherten Feuchtigkeit kann während der Anwendung von der Haut aufgenommen werden. Hierdurch kann der Feuchtigkeitshaushalt in der Haut optimiert werden.

Ein weiterer Vorteil des feuchtigkeitsregulierenden Mittels ist, dass es den Grundkörper stabilisieren kann, so dass auf die Verwendung zusätzlicher Verstärkungselemente verzichtet werden kann.

Erfindungsgemäß ist der mindestens eine Wirkstoff in einer Polymermatrix eingebettet. Besonders bevorzugt ist der mindestens eine Wirkstoff zumindest teilweise verkapselt. Die Verkapselung bzw. Einbettung des Wirkstoffs bietet den Vorteil, dass der mindestens eine Wirkstoff vor der Freisetzung und Anwendung in der Polymermatrix immobilisiert wird und vor äußeren Einflüssen wie Sauerstoff, Feuchtigkeit und/oder Temperaturschwankungen geschützt wird.

Weiter vorteilhaft an der Einbettung des Wirkstoffs in einer Polymermatrix ist, dass der mindestens eine Wirkstoff so besser vor Umwelteinflüssen geschützt wird als in einer Lösung, Creme oder Salbe. Dies ermöglicht eine längere Haltbarkeit. Die Einbettung in der Polymermatrix ist außerdem technisch einfacher, da die Wirkstoffe durch die Polymermatrix in ihrer Position fixiert sind, was die Einbettung in eine Emulsion, wie beispielsweise Creme, unnötig macht. Ein weiterer Vorteil ist, dass das Flächengebilde als Rollenware einfach gelagert und transportiert werden kann.

Darüber hinaus können mehrere Wirkstoffe voneinander separiert werden, was die Lagerung des Flächengebildes vereinfacht. Insbesondere kann eine unerwünschte Reaktion der Wirkstoffe untereinander verhindert und/oder eine zeitlich versetzte Freigabe der Wirkstoffe ermöglicht werden.

Erfindungsgemäß liegen die Fasern des textilen Flächengebildes und das feuchtigkeitsregulierende Mittel wenigstens teilweise stoffschlüssig miteinander verbunden vor. Hierdurch kann eine sehr gleichmäßig verteilte Feuchtigkeitsaufnahme- bzw. abgabekapazität erzielt werden. Dies führt zu einer gleichmäßigen Befeuchtung des Flächengebildes und somit zu einer gleichmäßigen Freisetzung und Verteilung des Wirkstoffs beispielsweise auf der Haut.

Bei einem stoffschlüssigen Verbinden zwischen feuchtigkeitsregulierendem Mittel und Fasern ist vorteilhaft, dass ein einlagiger Aufbau des Flächengebildes möglich ist. Günstig an einem einlagigen Aufbau ist, dass ein derartiges Flächengebilde besonders flexibel und beweglich ist.

Eine stoffschlüssige Verbindung zwischen dem feuchtigkeitsregulierendem Mittel und den Fasern des Flächengebildes kann beispielsweise durch eine geeignete Auswahl der Ausgangsmaterialien und/oder die Anwendung des erfindungsgemäßen Herstellungsverfahrens erfolgen.

Weiterhin vorteilhaft ist, dass durch die vom feuchtigkeitsregulierenden Mittel erzeugbare feuchte Atmosphäre das Eindringen des mindestens einen Wirkstoffs in die Haut bzw. dessen Aufnahme in der Haut unterstützt werden kann.

Unter Wirkstoff wird erfindungsgemäß ein kosmetischer Stoff und/oder ein pharmazeutischer Stoff verstanden, wobei die Begriffe kosmetischer und/oder pharmazeutischer Stoff erfindungsgemäß im herkömmlichen Sinne verstanden werden. Insbesondere werden unter kosmetischen Stoffen Stoffe oder Zubereitungen verstanden, die dazu bestimmt sind, äußerlich mit den verschiedenen Teilen des menschlichen Körpers, wie beispielsweise der Haut, dem Behaarungssystem, den Nägeln, den Lippen und intimen Körperregionen oder mit Zähnen, Schleimhäuten der Mundhöhle in Berührung, zu kommen. Diese Stoffe haben beispielsweise den Zweck, die zuvor genannten Körperteile zu reinigen, zu parfümieren, ihr Aussehen zu verändern, den Körpergeruch zu beeinflussen die zuvor genannten Körperteile zu schützen und/oder zu pflegen.

Unter pharmazeutischen Stoffen sind insbesondere Stoffe und Zubereitungen zu verstehen, die dazu bestimmt sind, durch Anwendung am oder im menschlichen oder tierischen Körper Krankheiten, Leiden, Körperschäden oder krankhafte Beschwerden zu heilen, zu lindern, zu verhüten oder zu erkennen.

Unter einem feuchtigkeitsregulierendem Mittel wird erfindungsgemäß ein Mittel verstanden, das Flüssigkeit aufnehmen, speichern und/oder abgeben kann. Besonders bevorzugte feuchtigkeitsregulierende Mittel können Flüssigkeit aufnehmen, speichern und abgeben. Erfindungsgemäß bevorzugt ist das feuchtigkeitsregulierende Mittel ein quellbares, bevorzugt flüssigkeitsquellbares, besonders bevorzugt wasserquellbares Material zu verstehen. Insbesondere ist das feuchtigkeitsregulierende Mittel ein Material, das unter Normalbedingungen, d.h. 20 °C, 1,013 bar das 10fache bis das 500fache, vorzugsweise das 20fache bis 300fache, besonders bevorzugt das 20fache bis 200fache seines Eigengewichts an Flüssigkeit, beispielsweise Wasser, aufnehmen kann. Das feuchtigkeitsregulierende Mittel eignet sich vorzugsweise zur Aufnahme von beliebigen Flüssigkeiten, beispielsweise Wasser; Rosenwasser; Alkohole, besonders bevorzugt Glycerin, Glykol; Öle, besonders bevorzugt Pflanzenöle, ganz besonders bevorzugt Arganöl, Avocadoöl, Jojobaöl, Mandelöl, Nachtkerzenöl, Olivenöl, Rosenöl, Sanddornöl, Traubenkernöl, Paraffinöl und deren Gemische. Bevorzugt ist das feuchtigkeitsregulierende Mittel wasserunlöslich.

Gemäß einer bevorzugten Ausführungsform der Erfindung liegen die Fasern zumindest teilweise vom feuchtigkeitsregulierenden Mittel umhüllt vor. Dies ermöglicht eine besonders homogene Flüssigkeits- bzw. Feuchtigkeitsregulation des Flächengebildes.

Erfindungsgemäß wird als feuchtigkeitsregulierendes Mittel ein Superabsorber eingesetzt. Superabsorber zeichnen sich dadurch aus, dass sie Flüssigkeit hervorragend binden und aufnehmen können. Es wird unter einem Superabsorber ein Polymer verstanden, das in der Lage ist, ein Vielfaches seines Eigengewichts - bis zum 500-fachen - an Feuchtigkeit bzw. Flüssigkeiten, vorzugsweise Wasser, aufzusaugen bzw. aufzunehmen, wobei es an Volumen zunimmt. Erfindungsgemäß bevorzugt sind Superabsorber, die bei Normalbedingungen vorzugsweise das 20fache bis 300fache, besonders bevorzugt das 20fache bis 200fache an Flüssigkeit, insbesondere Wasser, aufnehmen können. Die Quellfähigkeit des Superabsorbers kann bekanntermaßen beispielsweise durch Einstellung des Vernetzungsgrades reguliert werden. Vorzugsweise wird als Superabsorber erfindungsgemäß ein Copolymer aus Acrylsäure (Propensäure, H₂C=CH-COOH) und Natriumacrylat (Natriumsalz der Acrylsäure, H₂C=CH-COONa) eingesetzt, wobei das Verhältnis der beiden Monomere zueinander variieren kann. Zusätzlich kann ein so genannter Kernvernetzer (Core-Cross-Linker, CXL) der Monomerlösung zugesetzt werden, der die gebildeten langkettigen Polymermoleküle stellenweise untereinander durch chemische Brücken verbindet. Durch diese Brücken kann das Polymer wasserunlöslich werden. Vorteilhaft an der Verwendung von Superabsorbern ist, dass sie bei Eintritt von Feuchtigkeit gelieren können, wodurch dem Flächengebilde eine polsternde Wirkung verliehen wird.

Die Polymermatrix enthält bevorzugt Polymere ausgewählt aus der Gruppe bestehend aus: Polysacchariden, bevorzugt Gummi arabicum, Maltodextrinen, Alginaten, Stärken, Cellulosen oder Cellulosederivaten, bevorzugt Methylcellulosen, Ethylcellulosen, Hydroxypropylcellulosen, Carboxymethylcellulosen, sulfonierten Cellulosen, Celluloseacetaten; Pektinen; Poly(meth)acrylatpolymeren, besonders bevorzugt Polyacrylsäuren, Polyalkyl(meth)acrylaten, Polyaminoalkyl(meth)acrylaten, Polyammonioalkyl(meth)acrylaten; Polyvinylpyrrolidonen; Polystyrolsulfonaten, anderen Polyelektrolyten, besonders bevorzugt Polyvinylaminen, Polydiallyldimethylammoniumchloriden; Polyalkylenoxiden, besonders bevorzugt Polyethylenoxiden und Polypropylenoxiden; Polyvinylacetat und teilverseiftem Polyvinylacetat, Polyvinylalkohol, Polyacrylamiden, Polyurethanen, Proteinen, Gelatine, Ligninsulfonaten, deren Gemische und/oder deren Copolymere. Praktische Versuche haben gezeigt, dass sich diese Polymere besonders gut zur Immobilisierung des Wirkstoffs eignen.

Gemäß einer weiteren bevorzugten Ausführungsform ist die Polymermatrix auf den Fasern und zwischen den Fasern, angeordnet. Dies ermöglicht eine homogene Verteilung der Polymermatrix im Grundkörper.

Gemäß einer weiteren bevorzugten Ausführungsform ist die Polymermatrix im Wesentlichen auf zumindest einer Oberfläche des Flächengebildes angeordnet. Dies ermöglicht eine maximale Kontaktfläche des Wirkstoffes mit dem zu behandelnden Körper.

Gemäß einer weiteren bevorzugten Ausführungsform der Erfindung sind die Polymermatrix und der mindestens eine Wirkstoff so aufeinander abgestimmt, dass der mindestens eine Wirkstoff kontrolliert und/oder sukzessiv freigesetzt werden kann. Der mindestens eine Wirkstoff wird bevorzugt durch einen externen Stimulus schlagartig, allmählich und/oder sukzessiv freigesetzt. Durch die kontrollierte Wirkstofffreisetzung können Schwankungen in der Wirkstoffkonzentration verhindert werden.

Bei mehreren Wirkstoffen werden diese bevorzugt so ausgewählt, dass sie durch einen externen Stimulus gemeinsam freigesetzt und gemischt werden können, unmittelbar bevor sie in Kombination eine Wirkung entfalten können. Eine sukzessive Freisetzung hat den Vorteil, dass die Haut nicht auf einen Schlag mit einer Vielzahl an Wirkstoffen in Kontakt tritt, sondern dass die Wirkstoffe in einer für die Anwendung bevorzugten Reihenfolge freigesetzt werden können. Zunächst könnten ein oder mehrere Wirkstoffe freigesetzt werden, die die Haut quellen lassen, die Poren öffnen und/oder anderweitig die Haut vorbereiten. Im Anschluss daran könnte dann ein Wirkstoff oder mehrere zur Bräunung, Aufhellung, Straffung und/oder Klärung der Haut freigesetzt werden. Als letztes könnten schließlich noch ein Wirkstoff oder mehrere Wirkstoffe zur abschließenden Beruhigung der Haut freigesetzt werden.

Gemäß einer weiteren bevorzugten Ausführungsform ist der mindestens eine Wirkstoff so ausgewählt, dass die Freisetzung des Wirkstoffes in einem Zeitraum erfolgt, der kürzer als die Dauer der Anwendung auf der Haut ist, bevorzugter in weniger als zwei Tagen, höchst bevorzugt in weniger als acht Stunden, besonders bevorzugt in weniger als einer Stunde. Die Freisetzung könnte in einem Zeitraum von einer Sekunde bis zwei Tagen, bevorzugter von zehn Sekunden bis einem Tag, höchst bevorzugt von einer Minute bis acht Stunden, besonders bevorzugt von fünf Minuten bis einer Stunde, erfolgen.

Gemäß einer bevorzugten Ausführungsform der Erfindung werden Wirkstoff und polymere Matrix so aufeinander abgestimmt, dass der mindestens eine Wirkstoff durch Auflösung der Polymermatrix freigesetzt werden kann. Dies ermöglicht eine einfache und kontrollierte Freisetzung des Wirkstoffs zu einem bestimmten Zeitpunkt.

Weiter vorzugsweise wird die Polymermatrix so ausgewählt, dass die Freisetzung des Wirkstoffs durch Auflösen, thermische Behandlung, Bestrahlung oder durch Quellung der Polymermatrix erfolgen kann. Dabei werden die intermolekularen Polymer-Polymer-Wechselwirkungen durch Einfluss des Stimulus verringert und der mindestens eine Wirkstoff könnte aus der Polymermatrix bzw. deren Überresten heraus diffundieren.

Weiter vorzugsweise wird die Polymermatrix so ausgewählt, dass die Freisetzung des Wirkstoffs durch Behandeln der Polymermatrix mit einer Flüssigkeit erfolgen kann. Bevorzugt werden zur Auflösung der Polymermatrix Flüssigkeiten ausgewählt aus der Gruppe bestehend aus: Wasser; Alkohole, besonders bevorzugt Glykole, Glycerin; Öle und deren Gemische eingesetzt. Die Auflösung der Polymermatrix könnte bei einer Temperatur im Bereich von 5 bis 60 °C und einem Druck im Bereich von 800 bis 1200 mbar erfolgen. Die Auflösung der Polymermatrix findet bevorzugt bei einer Menge an Flüssigkeit im Bereich von 5 bis 1000 Gew.-% statt.

Weiter vorzugsweise wird die Polymermatrix so ausgewählt, dass die Freisetzung des Wirkstoffes durch thermische Behandlung bei einer Temperatur im Bereich von 15 bis 120 °C und bei einem Druck im Bereich von 800 bis 1200 mbar erfolgen kann. Bevorzugt erfolgt die thermische Behandlung in einem Zeitraum von einer Minute bis einer Stunde.

Weiter vorzugsweise wird die Polymermatrix so ausgewählt, dass die Freisetzung des Wirkstoffs durch eine Änderung des pH-Wertes erfolgen kann. Bevorzugt wird der pH-Wert in einem Bereich von 2 bis 10 eingestellt. Die pH-Wertänderung erfolgt bevorzugt bei einer Temperatur im Bereich von 15 bis 60 °C. Die pH-Wertänderung könnte durch Zugabe von Fruchtsäuren, alpha-Hydroxysäuren, beta-Hydroxysäuren und/oder deren Gemischen erfolgen.

Weiter vorzugsweise wird die Polymermatrix so ausgewählt, dass die Freisetzung des Wirkstoffs durch Bestrahlung, bevorzugt durch Bestrahlung mit UV-Licht erfolgen kann. Bevorzugt findet die Freisetzung des Wirkstoffs bei einer Strahlungsintensität im Bereich von 0,01 bis 20 W/cm² statt. Die Strahlungszeit könnte im Bereich von einer Sekunde bis 60 Sekunden liegen.

Wie bereits oben erläutert ist es erfindungsgemäß bevorzugt, dass das erfindungsgemäße Flächengebilde einlagig aufgebaut ist. Dennoch ist grundsätzlich möglich, dass der Grundkörper mindestens eine weitere Lage aufweist. Durch weitere Lagen kann die Stabilität des Grundkörpers erhöht werden.

Der mindestens eine Wirkstoff könnte so ausgewählt sein, dass er zumindest teilweise in menschliche Haut eindringen kann und/oder zur äußeren Anwendung geeignet ist und/oder einen optischen, riechbaren und/oder fühlbaren Effekt auf der Haut oder in der Haut hervorrufen kann. Dies ermöglicht eine lokale oder systematische transdermale Wirkstoffverabreichung. Bevorzugt ist der mindestens eine Wirkstoff so ausgewählt, dass er in die oberen Hautschichten, bevorzugter in die Epidermis und Dermis eindringt. Dort kann der mindestens eine Wirkstoff die volumengebenden Zellen stimulieren, die Poren öffnen, eine Quellung der Haut verursachen und/oder die Produktion von Botenstoffen anregen, die die hauteigene Kollagenproduktion erhöhen. Ferner kann der mindestens eine Wirkstoff die Haut bräunen, aufhellen, straffen, klären und/oder reinigen.

Gemäß einer bevorzugten Ausführungsform ist der mindestens eine Wirkstoff ausgewählt aus der Gruppe bestehend aus: Pflanzenöl; Proteinen; Vitaminen, beispielsweise Vitamin E und/oder Vitamin A; Enzymen; Panthenol; Harnstoff; Lipiden; Fruchtsäuren; pflanzlichen Wirkstoffen, bevorzugt Aloe vera, Kamille, ganz besonders bevorzugt Bisabolol, Menthol, Capsaicin; tierischen Wirkstoffen; Hyaluronsäuren und deren Gemischen. Proteine eignen sich vorteilhafterweise als Wirkstoff aufgrund ihrer leicht straffenden Wirkung und ihrer Fähigkeit, Wasser zu binden. Capsaicin kann ein wärmendes Gefühl auf der Haut erzeugen. Menthol wird in der Regel aufgrund seiner kühlenden Wirkungen verwendet. Vitamine können in vielfältiger Weise den menschlichen Stoffwechsel beeinflussen. Beispielsweise kann als Vitamin Vitamin A eingesetzt werden, da es das Zellwachstum und die Kollagensynthese anregt. Enzyme können vorteilhafterweise bei der Entfernung von Ansammlungen toten Zellmaterials der Haut helfen, welche sich häufig als oberflächliche Faltenbildung zeigt oder schlicht das Abfließen von Talg verhindern, was dann in Hautunreinheiten bis hin zu entzündlichen Prozessen enden kann. Ein Vorteil an Lipiden ist, dass sie hervorragend hautverträglich sind. Lipide können den Lipidmantel der Haut unterstützen und können somit den transepidermalen Wasserverlust verringern, vor Austrocknung schützen und kleinere Hautvertiefungen ausgleichen. Fruchtsäuren eignen sich hervorragend zur Narben- und Aknebehandlung und zur Behandlung von Pigmentflecken und Altersfältchen. Kamille und Bisabolol wirken vorteilhafterweise beruhigend auf die Haut und endzündungshemmend. Panthenol erhöht das Feuchthaltevermögen der Haut und hat juckreizlindernde, entzündungshemmende und wundheilungsfördernde Wirkung. Aloe vera könnte verwendet werden, da es in der Lage ist, in die tieferen Hautschichten einzudringen. Hyaluronsäure wird bevorzugt zur Faltenbeseitigung verwendet.

Weiterhin bevorzugt beträgt die Konzentration des Wirkstoffs von 0,01 bis 70,00 Gew.-% bezogen auf das Gesamtgewicht von Polymermatrix und Wirkstoff. Die Konzentration kann für unterschiedliche Wirkstoffe in weiten Bereichen variieren. Bevorzugt wird Hyaluronsäure in einer Konzentration von 0,1 bis 0,5 Gew.-%, Bisabolol in einer Konzentration von 0,8 Gew.-%, Panthenol in einer Konzentration von 1 bis 10 Gew.-%, Harnstoff in einer Konzentration von 2 bis 10 Gew.-%, Vitamin E in einer Konzentration von 1 bis 5 Gew.-%, eingesetzt. Die Konzentration für Pflanzenöle könnte in einem Bereich von 10 bis 70 Gew.-% liegen.

Bevorzugt ist die Polymermatrix selbsthaftend. Hieran ist vorteilhaft, dass keine zusätzliche Adhäsionsschicht oder ein Fixiermittel notwendig ist, um die Polymermatrix - und somit auch den mindestens einen Wirkstoff - im Flächengebilde zu fixieren. Dies ermöglicht das Aufbringen und die Fixierung der mindestens einen Wirkstoff enthaltenden Polymermatrix in einem Schritt.

Das Flächengebilde könnte im trockenen Zustand ein Flächengewicht, gemessen nach DIN-Norm ISO 9073-1, von 0,001 bis 40 g/m², bevorzugt von 0,001 bis 20 g/m², besonders bevorzugt von 0,001 bis 15 g/m², aufweisen. Die erfindungsgemäßen Flächengebilde zeichnen sich dadurch aus, dass sie ein geringes Gewicht mit einer hervorragenden mechanischen Festigkeit kombinieren können.

Bevorzugt weist das Flächengebilde die zwei- oder dreidimensionale Struktur eines Gesichts, eines Dekolletés, einer Hand, eines Arms, eines Fußes, eines Beines oder eines sonstigen Körperteils auf und/oder passt sich der Struktur dieser Körperteile an. Dies kann zu einer hervorragenden Passgenauigkeit und Anschmiegsamkeit des Flächengebildes an die zuvor genannten Körperteile führen, wodurch ein angenehmes Tragegefühl der Substratoberfläche beispielsweise auf der Haut entsteht. Ferner kann durch die Passgenauigkeit der Okklusionseffekt verstärkt werden.

Das textile Material könnte als Vlies oder Vliesstoff ausgestaltet sein. Dies ist vorteilhaft, da ein besonders flacher Aufbau realisiert werden kann und das Flächengebilde elastisch sein kann. Hierdurch kann der Tragekomfort des Flächengebildes während der Anwendung erhöht werden.

Bevorzugt wird ein Vliesstoff eingesetzt. Dieser könnte mechanisch, chemisch und/oder thermisch verfestigt sein. Ein mechanisches Verfestigen kann mittels Nadeltechnik oder durch ein Verschlingen von Fasern der Vliesstofflage mittels Wasserstrahlen und/oder Luft erfolgen. Hierdurch kann ein besonders weiches Produkt mit einem niedrigen Flächengewicht hergestellt werden.

Gemäß einer bevorzugten Ausführungsform enthält der Grundkörper Fasern, ausgewählt aus der Gruppe bestehend aus: Polyolefinen-, bevorzugt Polyphenylensulfid-; Polyester-, bevorzugt Polyethylenterephthalat-, Polybutylenterephthalat-; Polyamid-, bevorzugt Polyamid 66 (Nylon®)-, Polyamid 60 (Perlon®)-; Polyvinylchlorid-; Polyacrylnitril-; Polyimid-; Polytetraflourethylen (Teflon®)-; Aramid-; Woll-; Baumwoll-; Seide-; Hanf-; Bambus-; Kenaf-; Sisal-; Cellulose-; Soja-; Flachs-; Kieselgel-; Viskosefasern und deren Gemische. Als weitere Fasern können auch Bikomponentenfasern und/oder Mehrkomponentenfasern, gelierend oder nicht-gelierend, verwendet werden. Dabei können die Bikomponentenfasern und/oder Mehrkomponentenfasern mit geometrischen Formen wie "Core shell", "Side-by-Side", "Pie- oder Orange-type", "Matrix with fibrils" eingesetzt werden. Die Bikomponentenfasern und/oder Mehrkomponentenfasern können zur thermischen Verfestigung der Vliese genutzt werden. Bei Erwärmung dieser Fasern kann eine thermische Bindung des Vlieses erfolgen. Beispielsweise schmilzt bei einer Core shell-Faser der shell-Anteil und verfestigt somit das Vlies. Als Bikomponentenfasern und/oder Mehrkomponentenfasern können Fasern aus Polyethylen/Polypropylen, Polyethylen/Polyester, Co-Polyester/Polyethylenterephthalat, Polyamid6/Polyamid6.6, Polybutylenterephthalat/Polyethylenterephthalat eingesetzt werden.

Besonders bevorzugt enthält der Grundkörper Fasern, ausgewählt aus der Gruppe bestehend aus: Polyethylen-, Polypropylen-, Polyamid-, Polyester, Poly-p-phenylenterephthalamid-, Poly-m-phenylenterephthalamid-, Baumwoll-, Viskosefasern und deren Gemische. Praktische Versuche haben gezeigt, dass ein Flächengebilde aufgebaut aus den zuvor genannten Fasern besonders hautverträglich ist.

Wie oben erläutert ist ein wesentlicher Vorteil des Flächengebildes, dass es einen Okklusionseffekt erzeugen kann. Nichtsdestotrotz kann es für mache Anwendungen vorteilhaft sein, wenn der Grundkörper Lüftungsöffnungen aufweist. Die Lüftungsöffnungen könnten aufgrund der Faserstruktur in Form von Poren enthalten sein. Besonders bevorzugt ist ein Grundkörper mit einer Porosität gem. ISO 8971-4 im Bereich von 50 bis 95 %, bevorzugt im Bereich von 80 bis 90 %. Weiterhin denkbar ist, dass die Lüftungsöffnungen durch das Formen von Aussparungen und/oder Durchgängen eingebracht werden können. Bei Berührung des Flächengebildes mit Wasser können sich diese Lüftungsöffnungen zumindest teilweise verschließen. Dies ermöglicht einen Flüssigkeitsstau in den oberen Hautschichten und die Wirkung des Wirkstoffs kann erhöht werden.

Bevorzugt weist das Flächengebilde nur auf einer Seite einen Wirkstoff auf. Hierdurch wird die Effizienz der Applizierung des Wirkstoffs gesteigert, da das Flächengebilde nur auf einer Seite mit einem Wirkstoff versehen werden muss. Das Flächengebilde könnte eine eindeutig für den Anwender erkennbare wirkende Seite und eine Rückseite aufweisen. Die Rückseite kann einen trockenen Griff aufweisen, wodurch das Benutzen des Flächengebildes für kosmetische Anwendungen, beispielsweise als Gesichtsmaske auch über einen längeren Zeitraum, z.B. über Nacht, ermöglicht wird.

Die Erfindung umfasst auch ein Verfahren zur Herstellung eines Flächengebildes umfassend die folgenden Verfahrensschritte:
a) Behandeln eines Grundkörpers aus einem textilen Material, welches Fasern aufweist, mit einer Vorstufe eines feuchtigkeitsregulierenden Mittels in Form einer Mischung enthaltend ein polymerisierbares Monomer oder Oligomer, sowie ein Benetzungsmittel und einen Initiator,
b) Polymerisation des Monomers oder Oligomers zu einem feuchtigkeitsregulierenden Mittel und
c) Versehen des Grundkörpers (2) mit mindestens einem Wirkstoff und einer Polymermatrix derart, dass der mindestens eine Wirkstoff in der Polymermatrix eingebettet wird und/oder
d) Versehen des Grundkörpers (2) mit mindestens einem in einer Polymermatrix eingebetteten Wirkstoff.

Überraschend wurde festgestellt, dass durch die Verwendung eines Benetzungsmittels die Oberflächenspannung einer Vorstufe eines feuchtigkeitsregulierenden Mittels in Form einer Mischung enthaltend ein polymerisierbares Monomer oder Oligomer und einen Initiator derart beeinflusst werden kann, dass ein stoffschlüssiges Verbinden des durch Polymerisation gebildeten feuchtigkeitsregulierenden Mittels mit den Fasern erfolgen und das feuchtigkeitsregulierende Mittel stoffschlüssig mit den Fasern verbunden werden kann.

Somit kann erfindungsgemäß zum Verbinden des feuchtigkeitsregulierenden Mittels mit den Fasern, die im Grundkörper enthalten sind, auf die Verwendung eines Klebers, Bindemittels und/oder Haftvermittlers zur Fixierung des feuchtigkeitsregulierenden Mittels verzichtet werden. Dies ist insbesondere bei körpernah eingesetzten Produkten von Vorteil, da auf zusätzliche Chemikalien verzichtet werden kann.

Mit dem erfindungsgemäßen Verfahren kann das feuchtigkeitsregulierende Mittel zwischen die Fasern und auf die Fasern eingebracht und fest mit diesen verbunden werden. Hierdurch kann eine gezielte Steuerung der Feuchtigkeitsaufnahme bzw. -abgabe und Quellung des feuchtigkeitsregulierenden Mittels im Flächengebilde erfolgen und die im feuchtigkeitsregulierenden Mittel gespeicherte Feuchtigkeit bzw. Flüssigkeit kann einen Okklusionseffekt bewirken, wodurch eine Wirkstoffpenetration erleichtert wird.

Unter einem Benetzungsmittel sind natürliche oder synthetische Stoffe zu verstehen, die in Lösung oder in Mischungen Oberflächenspannungen von Wasser oder anderen Flüssigkeiten herabsetzen, so dass diese besser in Oberflächen fester Körper eindringen und sie unter Verdrängung von Luft durchtränken und benetzen können.

Bevorzugt wird ein Benetzungsmittel ausgewählt aus der Gruppe bestehend aus: Glycerin, Propylenglykol, Sorbitol, Trihydroxystearin, Phospholipide, Ethylenoxid/Fett-Alkoholether, Ethoxylaten des Propylenoxids mit Propylenglykol, Ester des Sorbitols und des Glycerins und deren Gemische.

Besonders bevorzugt wird als Benetzungsmittel eine Verbindung der folgenden Formel

RO(CH₂CH₂O)ₓH,

verwendet, wobei R ein linearer oder verzweigter Alkylrest ist und wobei x= 4 bis 11 ist, bevorzugter 6 bis 10, besonders bevorzugt 6 bis 9. Abhängig vom Herstellungsverfahren ergibt sich bei kommerziellen Produkten immer eine natürliche Verteilung mehrerer Kettenlängen, die nebeneinander vorliegen. Praktische Versuche haben gezeigt, dass bei der Verwendung eines derartigen Benetzungsmittels die Oberflächenspannung der Mischung besonders effektiv herabgesetzt wird, wodurch das Eindringen der Mischung in den Grundkörper erleichtert wird. Dies führt zu einer ausgezeichneten Haftung zwischen dem feuchtigkeitsregulierenden Mittel und den Fasern, welche im Grundkörper enthalten sind.

Unter Alkylrest wird eine gesättigte aliphatische Kohlenwasserstoffgruppe mit 1 bis 30, bevorzugt mit 3 bis 20, bevorzugter mit 4 bis 17 und besonders bevorzugt mit 6 bis 11 Kohlenstoffatomen verstanden. Eine Alkylgruppe kann linear oder verzweigt sein und ist wahlweise mit einem oder mehreren aliphatischen, bevorzugt gesättigten, Kohlenwasserstoffgruppen mit 1 bis 4 Kohlenwasserstoffen substituiert.

Praktische Versuche haben gezeigt, dass bei einem Anteil des Benetzungsmittels bezogen auf die Gesamtmenge der Mischung im Bereich von 0,1 bis 5 Gew.-%, bevorzugter von 1 bis 4 Gew.-%, besonders bevorzugt von 1,5 bis 3,5 Gew.-% eine besonders gleichmäßige und homogene Benetzung des Grundkörpers erfolgt.

Besonders gute Ergebnisse bzgl. der Benetzung des Grundkörpers wurden erzielt bei Zugabe eines Benetzungsmittels, das eine Oberflächenspannung gemäß DIN 55660 der Mischung im Bereich von 10 bis 72 dyn einstellt, bevorzugt im Bereich von 15 bis 72 dyn, besonders bevorzugt im Bereich von 20 bis 72 dyn.

Gemäß einer bevorzugten Ausführungsform der Erfindung wird der Mischung mindestens ein Vernetzer zugegeben.

Vernetzung umfasst Reaktionen, bei denen eine Vielzahl einzelner Makromoleküle zu einem dreidimensionalen Netzwerk verknüpft werden. Die Verknüpfung kann entweder direkt beim Aufbau der Makromoleküle oder durch Reaktionen an bereits bestehenden Polymeren erreicht werden.

Durch den Prozess der Vernetzung können sich die Eigenschaften der vernetzten Stoffe verändern. Die Veränderung nimmt mit steigendem Vernetzungsgrad zu. Bei dem Vernetzungsgrad handelt es sich um ein quantitatives Maß zur Charakterisierung von polymeren Netzwerken. Der Vernetzungsgrad wird berechnet als Quotient aus einer Molzahl vernetzter Grundbausteine und einer Molzahl insgesamt vorhandener Grundbausteine im makromolekularen Netzwerk. Er wird entweder als dimensionslose Zahl oder in Prozent (Stoffmengenanteil) angegeben.

Der eingesetzte Vernetzer kann die Monomere bzw. Oligomere stellenweise untereinander durch chemische Brücken verbinden bzw. vernetzen. Diese Brückenbildung kann die Wasserunlöslichkeit des feuchtigkeitsregulierenden Mittels erhöhen. Beim Eindringen von Flüssigkeit in das feuchtigkeitsregulierende Mittel quillt dieses auf und strafft auf molekularer Ebene dieses Netzwerk. Somit kann Feuchtigkeit bzw. Flüssigkeit im Flächengebilde gespeichert werden und der Okklusionseffekt wird begünstigt.

Gemäß einer bevorzugten Ausführungsform der Erfindung weist der Vernetzer mindestens zwei reaktive funktionelle Gruppen auf, die mit funktionellen Gruppen der polymerisierbaren Monomere oder Oligomere während der Polymerisation reagieren können.

Vorteilhafterweise weist der Vernetzer mindestens eine Olefin-, Carboxyl-, und/oder Carboxylatgruppe auf. Bevorzugt sind die Vernetzer ausgewählt aus der Gruppe bestehend aus: Ethylenglykolbisacrylat, Diethylenglykoldimethacrylat, Polyethylenglykoldimethacrylat, Propylenglykoldimethacrylat, Polypropylenglykoldimethacrylat, Tetramethylolmethantrimethacrylat, N-Methylolacrylamid, Glycerintrimethacrylat, Glycidylmethacrylat, N,N'-Methylenbismethacrylamid, Diallylmaleat, Diallylphthalat, Diallylterephthalat, Triallylcyanurat, Triallylisocyanurat, Triallylphosphat, Dipentaerythritolhexaacrylat, Polyethylenglykoldiglycidylether, Di- oder Polyglycidylether von aliphatischen, polyvalenten Alkoholen, Ethylenglykolglycidylether, Myrcene und deren Gemische.

Besonders bevorzugte Vernetzer sind Triethylenglykoldimethacrylat, Ethylendimethacrylat, 1,1,1-Trimethylpropantriacrylat, 1,3,5-Tri-allyl-1,3,5-triazin-2,4,6(1H,3H, 5H)-trion, 1,3-Butandioldimethacrylat, 1,4-Butandioldimethacrylat, Ethylenglykoldimethacrylat, Trimethylolpropantrimethacrylat, N,N'-Methylendiacrylamid, N,N'-Methylenbismethacrylamid und deren Gemische. Diese Vernetzer eignen sich besonders zu einer gezielten Steuerung der Aufnahmekapazität des feuchtigkeitsregulierenden Mittels sodass ein angenehmes Tragegefühl auf der Haut hervorgerufen werden kann.

Bevorzugt wird ein Vernetzungsgrad im Bereich von 0,1 bis 10 %, bevorzugt von 1 bis 5 %, eingestellt. Durch einen hohen Vernetzungsgrad kann die Aufnahmekapazität des feuchtigkeitsregulierenden Mittels beschränkt werden.

Gemäß einer bevorzugten Ausführungsform der Erfindung beträgt der Anteil des Vernetzers bezogen auf die Gesamtmenge des Monomeranteils 0,01 bis 10,00 Gew.-%, bevorzugter 0,05 bis 5,00 Gew.-%, besonders bevorzugt 0,10 bis 3,00 Gew.-%. Ein solcher Anteil des Vernetzers hat sich als in der Regel besonders geeignet zur Steuerung der Aufnahmekapazität des feuchtigkeitsregulierenden Mittels auf erwünschte Werte erwiesen.

Gemäß einer weiteren bevorzugten Ausführungsform ist das polymerisierbare Monomer oder Oligomer ausgewählt aus der Gruppe bestehend aus: monoethylenisch ungesättigte Mono-Carbonsäuren, bevorzugt Acrylsäure, Methacrylsäure, Maleinsäure, Fumarsäure; Crotonsäure, Sorbinsäure, Itaconsäure, Zimtsäure; monoethylenisch ungesättigte Polycarbonsäureanhydride, bevorzugt Maleinsäureanhydrid; Carbonsäuresalze, bevorzugter wasserlösliche Salze, besonders bevorzugt Alkali-, Ammonium- oder Aminsalze; monoethylenisch ungesättigte Mono- oder Polycarbonsäuren, bevorzugt Natriummeth-, Trimethylaminmeth-, Triethanolaminmeth-, Natriummaleat, Methylaminmaleat; Sulfonsäuren, bevorzugt aliphatische oder aromatische Vinylsulfonsäuren, besonders bevorzugt Vinyl-, Allyl-, Vinyltoluol-, Styrol-, Methacrylsulfonsäuren; 2-Hydroxy-3-methacryloxypropylsulfonsäure; Sulfopropylmethacrylat, Sulfonsäuresalze, bevorzugt Alkali-, Ammonium-, Aminsalze von sulfonsäuregruppenhaltigen Monomeren oder Oligomeren; Hydroxyverbindungen, bevorzugt monoethylenisch ungesättigte Alkohole, monoethylenisch ungesättigte Ether oder Ester von Polyolen, besonders bevorzugt Methallylalkohol, Alkylenglykolen, Glycerin, Polyoxyalkylenpolyolen, Hydroxyethylmethacrylat, Hydroxypropylmethacrylat, Triethylenglykolmethacrylat, Polyoxyethylenoxypropylenglykolmonomethallylether, wobei die Hydroxygruppen etherifiziert oder esterifiziert sind; Amide, bevorzugt Vinylform-, Methacryl-, N-Alkylmeth-, N,N-Dialkylmethacryl-, N-Hydroxyalkylmethacryl-, N-Hexylacryl-, N,N-Dimethylacryl-, N,N'-Di-n-propylacryl-, N-Methylolmethacryl-, N-Hydroxyethylmethacryl-, N,N-Dihydroxyethylmethacrylamid, Vinyllactame, besonders bevorzugt N-Vinylpyrrolidon; Aminoverbindungen, bevorzugt aminogruppenhaltige Ester, monoethylenisch ungesättigter Mono- oder Dicarbonsäuren, heterocyclische Vinylverbindungen, besonders bevorzugt Dialkylaminoalkyl-, Dihydroxyalkylaminoalkyl-, Morpholinoalkylester; quartäre Ammoniumsalze, bevorzugt N,N,N-Trialkyl-N-methacryloyloxyalkylammoniumsalze, besonders bevorzugt N,N,N-Trimethyl-N-methacryloyloxyethylammoniumchlorid, N,N,N-Triethyl-N-methacryloyloxyethylammoniumchlorid, 2-Hydroxy-3-methacryloyloxypropyltrimethylammoniumchlorid, besonders bevorzugt Dimethylaminoethylmethacrylat, Diethylaminoethylmethacrylat, Morpholinoethylmethacrylat, Dimethylaminoethylfumarat und deren Gemische.

Bevorzugt beträgt der Anteil des Monomers oder Oligomers bezogen auf die Gesamtmenge der Mischung 3 bis 80 Gew.-%, bevorzugter 5 bis 70 Gew.-%, besonders bevorzugt 10 bis 50 Gew.-%. Praktische Versuche haben ergeben, dass bei diesem Monomer- bzw. Oligomeranteil die Aufnahmekapazität des feuchtigkeitsregulierenden Mittels, insbesondere von Wasser, besonders günstig ist und das Flächengebilde eine gute Stabilität zeigt für eine Anwendung auf der Haut.

Als Initiatoren werden Stoffe bezeichnet, die der Mischung, enthaltend Monomere bzw. Oligomere und Benetzungsmittel, zugegeben werden, um die gewünschte Polymerisation zu ermöglichen und zu starten bzw. zu initiieren.

Zweckmäßigerweise werden als Initiatoren wasserlösliche Azoverbindungen; Redoxsysteme; Peroxycarbonsäuren; Peroxycarbonsäureester; Thioxanthen; Thioamine; Ketonperoxide; Hydroperoxide; Dicarbonate; Oxalate; Nitrile, bevorzugt Valeronitrile; Anisoine; Benzophenone; Acetophenone; Antrachinone; Benzolchromtricarbonyle; Benzoine; Benzoinether; Benzile; Benzilketale; 4-Benzoylbiphenyle; Phenylpropandiole; 10,11-Dihydro-5H-dibenzo[a,d]cyclohepten-5-one; Diphenyl(2,4,6-Trimethylbenzoyl)phosphinoxide; 2-Hydroxy-2-methylpropiophenone; 4'-Ethoxyacetophenone; Ethylanthrachinone; 1-Hydroxycyclohexylphenylketone, 2-Methyl-4'-(methylthio)-2-morpholinopropiophenone, Phenanthrenchinone, 4-Phenoxyacetophenone; α-Hydroxyketone; Phenylglyoxylate; Benzyldimethylketale; α-Aminoketone; 2,5-Dimethyl-2,5-dihydroperoxy-hexan; 1,3-Di-(2-hydroxyperoxyisopropyl)-benzol; Monoacylphosphine; Bisacylphosphine; Phosphinoxide; Metalloccene; Peroxide; Persulfate; Chlorite; lodsalze und/oder Hypochlorite eingesetzt; bevorzugt 2,2'-Azobis[2-(2-imidazolin-2-yl)propandihydrochlorid; Azobis(2-Amidinopropan)dihydrochlorid); Azo-bis-cyanopentansäure; 4-Benzoyl-N,N,N-trimethylbenzolmethanaminiumchlorid; 2-Hydroxy-3-(4-benzoylphenoxy)-3-N,N,N-trimethyl-1-propanaminiumchloridmonohydrat; 2-Hydroxy-3-(3,4-dimethyl-9-oxo-9H-thioxanthon-2-yloxy)-N,N,N-trimethyl-1-propanaluminiumiumchlorid; 2-Hydroxy-1-[4-(hydroxyethoxy)phenyl]-2-methyl-1-propanon; 2-Hydroxy-2-methyl-1-phenylpropan-1-on; 4-Benzoyl-N,N-dimethyl-N-[2-(1-oxo-2-propenly)oxy]ethylbenzolmethanaminiumchlorid; 1-[4-(2-Hydroxyethoxy)-phenyl]-2-hydroxy-2-methyl-1-propan-1-on; 2,2'-Azobis(4-Methoxy-2,4-dimthylvaleronitril); Antrachinon-2-sulfonsäure Natriummonohydrate; Bis(2,4,6-trimethylbenzoyl)-phenylphosphinoxide; Benzoamine; Benzoinethylether; Benzoinmethylether; Benzoinisobutylether; 3,3',4,4'-Benzophenontetracarboxyldianhydrid; 4-Phenylbenzophenon; 2-Benzyl-2-(dimethylamino)-4'-morpholinobutyrophenon; 4,4'-Bis(diethylamino)benzophenon; 4,4'-Bis(dimethylamino)benzophenon, 4,4'Dimethylbenzil; 2,5-Dimethylbenzophenon; 3,4-Dimethylbenzophenon; 3'-Hydroxyacetophenon; 4'-Hydroxyacetophenon, 3-Hydroxybenzophenon; α, α-Dimethoxy-α-phenylacetophenon; 4-Hydroxybenzophenon; 2-Methylbenzophenon; Dialkoxyacetophenone; α-Hydroxyalklyphenone; α-Aminoalkylphenon; 4,4'-Dihydroxybenzophenone; 2,2-Dimethoxy-2-phenylacetophenon; 4-(Dimethylamino)benzophenon, 3-Methylbenzophenon, 1-Hydroxycyclohexylphenylketon; 2-Hydroxy-2-methylpropiophenon; 2-Hydroxy-2-methyl-propiophenon; 4-Dimethylaminobenzophenon; 2,2-Diethoxy-2-phenylacetophenon; 2,2-Diethoxyacetophenon; Methylbenzoylformat; Oxy-phenyl-essigsäure-2-[2-oxo-2-phenyl-acetoxy-ethoxy]-ethylester; Oxy-phenyl-acrylat-2-[2-hydroxy-ethoxy]-ethylester; 2-Chlorthioxanthen-9-one; 2-Benzyl-2-(dimehylamino)-1-[4-(4-morpholinyl)phenyl]-1-butanon; 2-Methyl-1-[4-(4-morpholinyl)phenyl]-1-propanon; Diphenyl-(2,4,6-trimethylbenzoyl)-phosphinoxid; Phenyl-bis-(2,4,6-Trimethyl)-benzoyl-phospinoxid; Ferrocen; Titanocen; Bis-η⁵-2,4-cylopentadien-1-yl)-bis-[2,6-difluoro-3-(1H-pyrro-1-yl)-phenyl]titan; (4-Methylphenyl)-[4-(2-methylpropyl-(4-methylphenyl)-[4-(2-methylproplyl)phenyl]-iodoniumhexafluorophosphat; Ammoniumpersulfat; Kaliumpersulfat; Kampferchinon; Dibenzocycloheptadienon; Hydroxyacetophenone; Thioxanthen-9-one; 4,4'-Dimethylbenzil; 2-Ethylanthrachinon; Acrylphosphinoxid; 2-Methylbenzoylformat; Didecanoylperoxid; Dilaurylperoxid; Dibenzoylperoxid; Di-(2-ethyl)-peroxidicarbonat; Dicylohexylperoxidicarbonat; Di-(4-tert.-butyl)-cylcohexylperoxidicarbonat; Diacetylperoxodicarbonat; Dimyristylperoxodicarbonat; Di-tert-butylperoxyoxalat; 2,2-Azobis(2,4-dimethylvaleronitril); 2,2-Azobis(4-methoxy-2,4-dimethylvaleronitril); 2,2'-Azobis(2-methylbutyronitril); 2,2'-Azobis(N-(2-propenyl)-2methylpropionamid; Dimethyl-2,-2'-Azobis(2-methylpropionat); Dimethyl-2,2'-azoisobutyrat; 1-Hydroxy-cyclohexylphenylketone; Peroxycarbonsäureester, hergestellt aus Pivalinsäure, Neodecansäure, 2-Ethylhexansäure, tert-Butylhydroperoxid, tert.-Amylhydroperoxid und/oder Cumolhydroxid; tert.-Amylhydroperoxid; Cumolhydroperoxid; Diacylperoxid; Wasserstoffperoxid; 2-Di(3,5,5-trimethylhexenoyl)peroxid; Hydroxy- und/oder tert.-Butylperoxid, insbesondere Bis(2,4,6-trimethylbenzoyl)-phenylphosphinoxide, 1-Hydroxycyclohexylphenylketone, Benzophenone und/oder 1-[4-(2-Hydroxyethoxy)-phenyl]-2-hydroxy-2-methyl-1-propan-1-one, eingesetzt.

Besonders bevorzugt ist der Initiator ausgewählt aus der Gruppe bestehend aus: N,N,N',N'-Tetramethylethylendiamin, Kaliumperoxodisulfat mit N,N,N',N'-Tetramethylethylendiamin, Ammoniumperoxodisulfat mit N,N,N',N'-Tetramethylethylendiamin, 1-Hydroxycyclohexylphenylketon, 1-Hydroxycyclohexylphenylketon mit Benzophenon, 2-Hydroxy-2-methyl-1-phenyl-1-propanon, 2-Hydroxy-1-[4-(2-hydroxyethoxy)phenyl]-2-methyl-1-propanon, Methylbenzoylformiat, Phenylglyoxylate, ganz besonders bevorzugt Oxophenylessigsäure-2-[2-(2-oxo-2-phenyl-acetoxy)-ethoxy]-ethylester, Bisacylphosphine, Phosphinoxide, 4,4'-Azobis(4-cyanovaleriansäure), 2,2'-Azobis[2-[1-(2-hydroxyethyl)-2-imidazolin-2-yl]propan]dihydrochlorid, 2,2'-Azobis[2-(2-imidazolin-2-yl)propan]disulfatdihydrat, 2,2'-Azobis(2-methylpropionamidin)dihydrochlorid, 2,2'-Azobis[N-(2-carboxyethyl)-2-methylpropionamidin]hydrat, 2,2'-Azobis{2-[1-(2-hydroxyethyl)-2-imidazolin-2-yl]propan}dihydrochlorid, 2,2'-Azobis[2-(2-imidazolin-2-yl)propan], 2,2'-Azobis(1-imino-1-pyrrolidino-2-ethylpropan)dihydrochlorid, 2,2'-Azobis{2-methyl-N-[1,1-bis(hydroxymethyl)-2-hydroxyethl]propionamid} und/oder 2,2'-Azobis[2-methyl-N-(2-hydroxyethyl)propionamid].

Praktische Versuche haben ergeben, dass sich Initiatoren, die in der Lage sind Protonen zu abstrahieren (Typ-II-Initiatoren), besonders gut für Polymerisationen des Monomers oder Oligomers zu dem feuchtigkeitsregulierenden Mittel eignen, die ohne Vernetzer durchgeführt werden. Gemäß einer bevozugten Ausführungsform werden Typ-II-Initiatoren eingesetzt, die in der Lage sind von den Fasern Protonen zu abstrahieren. Hieran ist vorteilhaft, dass eine besonders gute Anbindung des feuchtigkeitsregulierenden Mittels an die Fasern erreicht werden kann.

Wird die Polymerisation in Gegenwart eines Vernetzers durchgeführt, haben sich Typ-I-Initiatoren als besonders geeignet erwiesen, da mit ihnen eine besonders schnelle Polymerisation des Monomers oder Oligomers zu dem feuchtigkeitsregulierenden Mittel erzielt werden kann.

Der Anteil des Initiators bezogen auf die Gesamtmenge der Mischung könnte im Bereich von 0,1 bis 3 Gew.-%, bevorzugt von 0,5 bis 2 Gew.-%, besonders bevorzugt von 0,7 bis 1,5 Gew.-%, liegen.

Durch die in Verfahrensschritt b) aus den Monomeren bzw. Oligomeren durchgeführte Polymerisation könnte ein Superabsorber gebildet werden.

Zur Bildung der Mischung kann das Monomer bzw. Oligomer, bevorzugt in einer wässrigen Lösung, gelöst oder emulgiert werden. Der Wassergehalt in der Mischung könnte im Bereich von 20 bis 90 Gew.-%, bevorzugt im Bereich von 30 bis 80 Gew.-%, jeweils bezogen auf die Gesamtmenge der Mischung, liegen. Wenn der Vernetzer nicht löslich ist, kann er in emulgierter Form zugesetzt werden. Zusätzlich kann ein mit Wasser mischbares organisches Lösungsmittel zum Lösen oder Dispergieren des Vernetzens zugefügt werden. Anschließend könnten das Benetzungsmittel und der Initiator zugegeben werden.

Die Polymerisation findet bevorzugt im sauren pH-Bereich von 3 bis 6, besonders bevorzugt von 4,3 bis 5,5, statt. Unter diesen Bedingungen ist die Mischung besonders stabil.

Zum Neutralisieren des Acrylsäuremonomers bzw. der oben erwähnten sauren Monomere oder Oligomere könnte ein Hydroxid, bevorzugt ein Alkalimetallhydroxid, besonders bevorzugt Natrium-, Kalium- oder Lithiumhydroxid, Carbonat eines Alkalimetalls und/oder Ammoniumhydroxid verwendet werden. Natrium- oder Kaliumhydroxid werden aufgrund ihrer kommerziellen Verfügbarkeit, ihres Preises und ihrer Sicherheit besonders bevorzugt eingesetzt.

Das Behandeln des Grundkörpers mit der Mischung könnte durch Beschichten, Tauchen oder Sprühen erfolgen. Bevorzugt wird die Mischung mittels Beschichten aufgebracht, insbesondere mittels Rakeln. Beim Rakeln arbeitet üblicherweise ein Streichmesser gegen eine Unterlage, Walze, Tisch oder das Substrat selbst. Unter einer Rakel wird eine Streichvorrichtung verstanden. Diese könnte über die Gesamtbreite der Warenbahn fixiert werden. Das Aufbringen der Mischung könnte mit einem einseitigen Rollauftrag erfolgen. Hierdurch wird das Verfahren vereinfacht und es erfolgt eine gleichmäßige Verteilung der Mischung im Grundkörper.

Die Auftragsmenge der Mischung zum Beschichten, Tauchen oder Sprühen des Grundköpers kann in weiten Bereich variieren. Üblicherweise werden Mengen im Bereich von 10 bis 2500 g/m², bevorzugt von 50 bis 1200 g/m², auf die Fasern, welche im Grundkörper enthalten sind, eingebracht.

Nach dem Beschichten, Tauchen oder Besprühen des Grundkörpers kann dieses zwischen zwei Rollen und/oder Walzen abgequetscht werden. Praktische Versuche haben gezeigt, dass bei einem Abquetschdruck in einem Bereich von 1 bis 8 bar, bevorzugt in einem Bereich von 3 bis 5 bar, die Auftragsmenge optimal eingestellt werden kann und eine homogene Verteilung der aufgetragenen Mischung in den Fasern, welche im Grundkörper enthalten sind, erfolgt.

Anschließend könnte in einem nächsten Schritt die Polymerisation bzw. Härtung der Monomere bzw. Oligomere stattfinden, wodurch das feuchtigkeitsregulierende Mittel gebildet wird. Je nach eingesetztem Initiator und Reaktionsbedingungen kann die Polymerisation autokatalytisch, thermisch, durch Einwirken von ionisierender Strahlung oder mittels Plasma eingeleitet werden. Bevorzugt polymerisiert das Monomer bzw. Oligomer in Gegenwart von ultravioletter Strahlung.

Die UV-Härtung könnte unter Verwendung einer UV-Lampe erfolgen. Strahlungsintensität und -zeit richten sich nach der Zusammensetzung der Mischung und der Beschaffenheit des Grundkörpers. Besonders gute Ergebnisse werden bei einer Strahlungsintensität im Bereich von 40 bis 400 Watt/cm, bevorzugt im Bereich von 100 bis 250 Watt/cm bei einer Strahlungszeit im Bereich von 0,1 bis 120 Sekunden erzielt. Zweckmäßigerweise wird die UV-Härtung unter Vakuum oder in Gegenwart eines anorganischen Gases, bevorzugt Stickstoff, Helium oder Argon oder in Luft, durchgeführt.

Das thermische Härten könnte in einem Ofen, in Luft oder in einer inerten Atmosphäre oder unter Vakuum stattfinden. Denkbar ist auch, die aufgetragene Mischung in einem Trockner, wie einem Durchlufttrockner oder einem Infrarottrockner zu polymerisieren bzw. zu härten. Üblicherweise erfolgt die Polymerisation bzw. Härtung in einem Temperaturbereich von 40 bis 100 °C.

Im Anschluss an die Polymerisation könnten Verunreinigungen durch Waschen aus dem Flächengebilde entfernt werden. Das Waschen findet bevorzugt mit Wasser statt und kann kontinuierlich oder diskontinuierlich erfolgen. Hierdurch können auf einfache Art und Weise hautunverträgliche Chemikalien aus dem Flächengebilde entfernt werden.

Gemäß einer bevorzugten Ausführungsform findet im Anschluss an die Polymerisation ein Neutralisationsschritt statt. Hierzu könnte das Flächengebilde durch ein Neutralisationsbad mit einem pH-Wert im Bereich von 9 bis 14, bevorzugt im Bereich von 10 bis 14, besonders bevorzugt im Bereich von 12 bis 14, geführt werden.

Zum Neutralisieren können die bereits zuvor genannten Hydroxide, bevorzugt Alkalimetallhydroxid, bevorzugter Natrium-, Kalium- oder Lithiumhydroxid, ein Carbonat eines Alkalimetalls und/oder Ammoniumhydroxid, verwendet werden.

Nach der Härtung bzw. Polymerisation könnte die restliche Flüssigkeit durch weiteres Trocknen im Umluftofen oder mit Infrarotlampen entfernt werden. Bevorzugt wird lediglich eine Seite des Grundkörpers mit einem Wirkstoff versehen. Das Flächengebilde könnte eine eindeutig für den Anwender erkennbare wirkende, aktive Seite und eine Rückseite aufweisen. Hierdurch wird die Effizienz der Applizierung des Wirkstoffs gesteigert. Es können Materialkosten eingespart werden, da nur eine Seite den Wirkstoff aufweist.

Das Versehen des Flächengebildes mit dem in der Polymermatrix eingebetteten Wirkstoff könnte durch Fixierung der Polymermatrix mittels eines Binders erfolgen. Bevorzugt wird ein Binder eingesetzt ausgewählt aus der Gruppe bestehend aus: Polysacchariden, bevorzugt Gummi arabicum, Maltodextrinen, Alginaten, Stärken, Cellulosen oder Cellulosederivaten, bevorzugt Methylcellulosen, Ethylcellulosen, Hydroxypropylcellulosen, Carboxymethylcellulosen, sulfonierten Cellulosen, Celluloseacetaten; Pektinen; Poly(meth)acrylatpolymeren, besonders bevorzugt Polyacrylsäuren, Polyalkyl(meth)acrylaten, Polyaminoalkyl(meth)acrylaten, Polyammonioalkyl(meth)acrylaten; Polyvinylpyrrolidonen; Polystyrolsulfonaten, andere Polyelektrolyte, besonders bevorzugt Polyvinylaminen, Polydiallyldimethylammoniumchloriden; Polyalkylenoxiden, besonders bevorzugt Polyethylenoxiden und Polypropylenoxiden; Polyvinylacetat und teilverseiftem Polyvinylacetat; Polyvinylalkohol; Polyacrylamiden; Polyurethanen; Proteinen; Gelatine; Ligninsulfonaten; deren Gemische und/oder deren Copolymere. Besonders bevorzugt werden Binder eingesetzt aus der Gruppe bestehend aus: Polysaccharide, ganz besonders bevorzugt Alginat, Cellulose, Maltodextrin, Stärke und deren Gemischen. Diese sind besonders hautverträglich.

Der Anteil des Binders bezogen auf das Gesamtgewicht des Flächengebildes könnte im Bereich von 1 bis 300 g/m², bevorzugter von 10 bis 250 g/m², besonders bevorzugt von 20 bis 200 g/m², liegen.

Gemäß einer bevorzugten Ausführungsform könnte das Versehen des Grundkörpers mit dem in einer Polymermatrix eingebetteten Wirkstoff durch Anfeuchten und anschließendes Andrücken erfolgen. Hierbei ist vorteilhaft, dass keine weiteren Bindemittel, Kleber oder Haftmittel zur Fixierung des Wirkstoffes notwendig sind.

Das Anfeuchten könnte mit Wasser erfolgen. Das Aufbringen des Wirkstoffes auf das Flächengebilde könnte durch Aufrieseln erfolgen. Im Anschluss könnte der mindestens eine Wirkstoff zur besseren Fixierung angedrückt werden. Dies könnte mittels eines Kalanders geschehen. Ein Kalander ist ein System aus mindestens zwei aufeinander angeordneten beheizten und polierten Walzen, durch deren Spalten ein Flächengebilde, vorzugsweise ein Vliesstoff, hindurchgeführt werden kann. Es werden unterschiedliche Bauformen eines Kalanders unterschieden. Diese richten sich nach der Anordnung der einzelnen Kalanderwalzen in der Seitenansicht. Üblich sind die Bauformen L-, F-, I- und Z-Form.

Der mindestens eine Wirkstoff könnte als mikroverkapselter Wirkstoff auf den Grundkörper aufgebracht werden. Bei der Mikroverkapselung findet eine Einkapselung feindisperser flüssiger oder fester Phasen durch Umhüllung mit filmbildenden Polymeren statt, die sich nach Emulgierung und oder Grenzflächenpolymerisation auf dem einzuhüllenden Material niederschlagen. Die Mikroverkapselung des Wirkstoffes könnte mittels Sprühtrocknung einer Lösung oder einer Emulsion durch Verwendung von gelösten und dispergierten Polymeren als Polymermatrix bzw. Hüllbildner erfolgen. Bei der Sprühtrocknung wird die zuvor zugeführte Lösung oder Emulsion durch Zerstäubung der Flüssigkeit entfernt.

Bei der Sprühtrocknung wird üblicherweise die flüssige Lösung oder Suspension, die den Wirkstoff und die Polymermatrix bzw. den Hüllbildner enthält, durch Düsen oder mittels einer schnell rotierenden Zerstäuberscheibe zu einem feinen Nebel versprüht. Dem entstehenden Sprühkegel wird heiße Luft oder ein Inertgas (bei oxidationsempfindlichen Stoffen) von unten entgegengeführt oder parallel zum Sprühkegel geführt.

Die große, relative Oberfläche der Flüssigkeitströpfchen bewirkt einen effektiven und schnellen Wärme- und Stoffaustausch zwischen den beiden Phasen. Das Trockengut fällt als mehr oder weniger feines Pulver, als Granulat oder in Form kleiner Perlen nach unten und wird am Boden des Trockners durch mechanische Räumer, Transportschnecken oder dergleichen ausgetragen.

Bevorzugt enthält die Lösung oder Emulsion mindestens einen Wirkstoff und mindestens ein Polymer der Polymermatrix.

Gemäß einer bevorzugten Ausführungsform wird der mindestens eine Wirkstoff und die Polymermatrix oder die Polymere der Polymermatrix gelöst oder dispergiert in einem Lösungsmittel. Das Lösungsmittel ist bevorzugt ausgewählt aus der Gruppe, bestehend aus: Wasser, Ethanol, Isopropanol, Aceton, Methylethylketon, Ethylacetat, Dichlormethan, Pentan, Hexan, Heptan, Petrolether oder Diethylether. Beim Auftragen einer Lösung ist vorteilhafterweise kein Zusatz eines Emulgators nötig.

Im Falle einer Emulsion könnten der mindestens eine Wirkstoff unverdünnt verwendet oder in einem Basisöl gelöst werden. Bevorzugt wird als Basisöl ein Pflanzenöl verwendet, ausgewählt aus der Gruppe bestehend aus: Arganöl, Avocadoöl, Jojobaöl, Mandelöl, Nachtkerzenöl, Olivenöl, Rosenöl, Sanddornöl, Traubenkernöl oder Paraffinöl.

Die Wirkstoffzubereitung könnte in einer Wasserphase, in der die Polymermatrix oder die Polymere der Polymermatrix gelöst oder suspendiert sind, derart emulgiert sein, dass eine ÖI-in-Wasser-Emulsion entsteht.

Die Emulsion könnte zusätzlich einen Emulgator enthalten. Dieser unterstützt vorteilhafterweise den Emulgiervorgang und stabilisiert die Emulsion. Unter Emulgator wird ein Stoff verstanden, der durch Herabsetzung der Grenzflächenspannung oder aufgrund sterischer Wirkung die Grenzfläche stabilisiert. Der Emulgator ist bevorzugt ausgewählt aus der Gruppe, bestehend aus: Natriumstearoyllactylat, Sorbitanmonooleat, Sorbitanmonostearat, Sucrosemonostearat, Sorbitantristearat, Diacetylweinsäureestermonoglycerid, Sorbitanmonolaurat, Sucrosedistearat, Triglycerolmonostearat, Glycerolstearatzitrat, Polyethyleneglykol-20-Sorbitanmonolaurat, Polyethylenglykol-4-Sorbitanmonolaurat, Polyethylenglykol-20-Sorbitanmonopalmitat, Polyethylenglykol-20-Sorbitanmonostearat, Polyethylenglykol-4-Sorbitanmonostearat, Polyethylenglykol-20-Sorbitantristearat, Polyethylenglykol-20-Sorbitanmonooleat, Polyglyceryl-3-Methylglucosedistearate, Methylglucosesequistearat, Cetylalkohol, Cetylstearylalkohol, Lecithin oder modifiziertes Lecithin, besonders bevorzugt hydriertes Lecithin. Die Verwendung eines Emulgators hat den Vorteil, dass ein kostengünstiges Flächengebilde hergestellt werden kann, da Polymere mit emulgierenden Eigenschaften oft kostenintensiv sind.

Gemäß einer vorteilhaften Ausführungsform wird die Oberflächenenergie des Flächengebildes durch Corona- und/oder Plasmabehandlung erhöht. Dabei erfolgt die Corona- und/oder Plasmabehandlung bevorzugt derart, dass der Oberfläche eine Oberflächenenergie gemäß DIN 55660 von 40 bis 72 dyn, bevorzugter von 50 bis 72 dyn, ganz besonders bevorzugt von 55 bis 72 dyn, verliehen wird. Hierbei ist vorteilhaft, dass die Oberfläche hydrophil bzw. hydrophob ausgerüstet werden kann, ohne größere Mengen Chemikalien beizumengen. Diese Ausrüstung dient zur optimalen Regulierung des Feuchtigkeits- bzw. Flüssigkeitsgehalts im Flächengebilde. Dies ist insbesondere bei körpernah eingesetzten Produkten, wie ein enger Kontakt des Flächengebildes mit der Haut, von Vorteil.

Das Flächengebilde eignet sich hervorragend zur kosmetischen und/oder medizinischen Anwendung, da es eine sukzessive und kontrollierte Freisetzung und ein angenehmes Tragegefühl ermöglicht.

### Kurzbeschreibung der Zeichnung

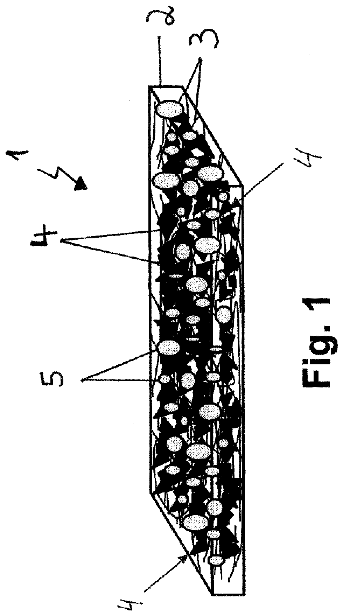

In der Zeichnung zeigt
- Fig. 1: ein Flächengebilde, umfassend einen Grundkörper aus einem textilen Material.

### Ausführung der Erfindung

Figur 1 zeigt ein Flächengebilde 1, umfassend einen Grundkörper 2 aus einem textilen Material, wobei der Grundkörper 2 Fasern 3 und einen Superabsorber 4 aufweist, wobei der Superabsorber 4 zwischen den Fasern 3 und auf den Fasern 3 angeordnet ist und wobei Menthol als Wirkstoff in einer Matrix 5 aus Gummi arabicum und Maltodextrin eingebettet ist.

Die Fasern 3 sind zumindest teilweise vom Superabsorber 4 umhüllt. Die Matrix 5 ist partikelförmig ausgestaltet.

Der Grundkörper 2, welcher in Figur 1 dargestellt ist, ist einlagig aufgebaut.

Das soeben beschriebene Flächengebilde kann gemäß dem folgenden Ausführungsbeispiel hergestellt werden:

### Ausführungsbeispiel 1:

Es werden 8,00 g Natriumhydroxid in 21,40 g Wasser gelöst und mit 20,60 g Acrylsäure versetzt. Anschließend wird die teilneutralisierte Acrylsäurelösung mit 1,00 g 1-[4-(2-Hydroxyethoxy)-phenyl]-2- hydroxy-2-methyl-1-propan-1-on, 2,00 g Heptylpolyethylenglykolether (C7H15O(CH2CH2O)6,5H und 45,00 g Wasser homogen vermischt. Der pH-Wert der Lösung beträgt ca. 4-5.

Der Lösung werden 2,0 g N,N'-Methylendiacrylamid hinzugegeben und diese wird 15 Minuten bei einer Temperatur von ca. 22 °C gerührt. Die Lösung wird durch Tauchen auf einen elastischen und drapierfähigen Vliesstoff aus Polyester mit einem Flächengewicht von 100 g/m² aufgetragen. Der imprägnierte Vliesstoff wird zwischen zwei Rollen abgequetscht und mit UV-Licht bestrahlt. Dazu wird eine UV-Lampe der Firma Dr. Hönle vom Typ Uvahand 250, 250 Watt pro Strahler, verwendet. Die Bestrahlungszeit beträgt 3 s. Der bestrahlte Vliesstoff wird mit Wasser gewaschen und vier Stunden bei 80 °C getrocknet.

Nach der Polymerisation hat das Gewicht des Vliesstoffes um ca. 5% zugenommen. Der Vernetzungsgrad der Beschichtung beträgt 4,3%. Der Restanteil monomerer Acrylsäure innerhalb des beschichteten Vliesstoffes kann mittels Extraktion mit Reinstwasser und anschließender quantitativer Messung von Acrylsäure mittels Ionenchromatographie bestimmt werden. Der Gehalt an Acrylsäure in 50 ml Reinstwasser nach Extraktion von 1,1 g für 1 h beträgt weniger als 30 ppm. Innerhalb einer Zeitspanne von 10 min in destilliertem Wasser nimmt das Gesamtgewicht des Vliesstoffes durch Quellung etwa um das 5fache zu.

## Patentansprüche

1. Flächengebilde (1), umfassend einen mit mindestens einem Wirkstoff versehenen Grundkörper (2) aus einem textilen Material, wobei der Grundkörper (2) Fasern (3) und einen Superabsorber als feuchtigkeitsregulierendes Mittel (4) aufweist, wobei der mindestens eine Wirkstoff in einer Polymermatrix (5) eingebettet ist, **dadurch gekennzeichnet, dass** die Fasern (3) zumindest teilweise stoffschlüssig mit dem feuchtigkeitsregulierenden Mittel (4) verbunden sind.

2. Flächengebilde nach Anspruch 1, **dadurch gekennzeichnet, dass** die Fasern (3) zumindest teilweise vom feuchtigkeitsregulierenden Mittel (4) umhüllt sind-

3. Flächengebilde nach Anspruch 1 oder 2, **dadurch gekennzeichnet, dass** die Polymermatrix (5) auf den Fasern (3), zwischen den Fasern (3), auf dem feuchtigkeitsregulierenden Mittel (4) und/oder im feuchtigkeitsregulierenden Mittel (4) angeordnet ist

4. Flächengebilde nach einem der vorangegangenen Ansprüche, **dadurch gekennzeichnet, dass** der mindestens eine Wirkstoff so ausgewählt ist, dass er kontrolliert und/oder sukzessiv freigesetzt werden kann.

5. Flächengebilde nach einem der vorangegangenen Ansprüche, **dadurch gekennzeichnet, dass** die Polymermatrix so ausgewählt ist, dass der mindestens eine Wirkstoff durch Auflösung der Polymermatrix (5) freigesetzt werden kann.

6. Flächengebilde nach einem der vorangegangenen Ansprüche, **dadurch gekennzeichnet, dass** der Grundkörper (2) mindestens eine weitere Lage enthält.

7. Flächengebilde nach einem der vorangegangenen Ansprüche, **dadurch gekennzeichnet, dass** der mindestens eine Wirkstoff so ausgewählt ist, dass er zumindest teilweise in menschliche Haut eindringen kann und/oder zur äußeren Anwendung geeignet ist und einen optischen, riechbaren und/oder fühlbaren Effekt auf der Haut oder in der Haut hervorrufen kann.

8. Flächengebilde nach einem der vorangegangenen Ansprüche, **dadurch gekennzeichnet, dass** der mindestens eine Wirkstoff ausgewählt ist aus der Gruppe bestehend aus: Pflanzenölen; Proteinen; Vitaminen; Enzymen; Lipiden; Fruchtsäuren; pflanzlichen Wirkstoffen; tierischen Wirkstoffen; Hyaluronsäuren und deren Gemische.

9. Flächengebilde nach einem der Ansprüche 3 bis 8, **dadurch gekennzeichnet, dass** die Polymermatrix (5) selbsthaftend ausgerüstet ist.

10. Flächengebilde nach einem der vorangegangenen Ansprüche, **dadurch gekennzeichnet, dass** das feuchtigkeitsregulierende Mittel (4) im trockenen Zustand ein Flächengewicht, gemessen nach DIN-Norm ISO 9073-1, von 0,001 bis 30 g/m², aufweist.

11. Flächengebilde nach einem der vorangegangenen Ansprüche, **dadurch gekennzeichnet, dass** das Flächengebilde (1) eine zwei- oder dreidimensionale Struktur eines Gesichts, eines Dekolletés, einer Hand, eines Arms, eines Fußes, eines Beines oder eines sonstigen Körperteils aufweist und/oder sich der Struktur dieser Körperteile anpasst.

12. Flächengebilde nach einem der vorangegangenen Ansprüche, **dadurch gekennzeichnet, dass** das textile Material als Vlies oder Vliesstoff ausgestaltet ist.

13. Verfahren zur Herstellung eines Flächengebildes nach einem der vorangegangenen Ansprüche, umfassend die folgenden Verfahrensschritte:
a) Behandeln eines Grundkörpers (2) aus einem textilen Material, welches Fasern aufweist, mit einer Mischung enthaltend eine Vorstufe eines feuchtigkeitsregulierenden Mittels (4) in Form eines polymerisierbaren Monomers oder Oligomers, sowie ein Benetzungsmittel und einen Initiator,
b) Polymerisation des Monomers oder Oligomers zu einem feuchtigkeitsregulierenden Mittel (4) und
c) Versehen des Grundkörpers (2) mit mindestens einem Wirkstoff und einer Polymermatrix derart, dass der mindestens eine Wirkstoff in der Polymermatrix eingebettet wird und/oder
d) Versehen des Grundkörpers (2) mit mindestens einem in einer Polymermatrix eingebetteten Wirkstoff.

14. Verfahren nach Anspruch 13, **dadurch gekennzeichnet, dass** der Mischung ein Vernetzer zugesetzt wird.

15. Verfahren nach Anspruch 14, **dadurch gekennzeichnet, dass** ein Vernetzungsgrad im Bereich von 0,1 bis 10 %, eingestellt wird.

16. Verwendung eines Flächengebildes nach einem der Ansprüche 1 bis 12 zur kosmetischen Behandlung.

## Claims

1. Sheet-like structure (1), comprising a main part (2) composed of a textile material that is provided with at least one active ingredient, said main part (2) having fibers (3) and a superabsorbent as moisture-regulating agent (4), wherein the at least one active ingredient is embedded in a polymer matrix (5), **characterized in that** the fibers (3) are at least partially integrally bonded to the moisture-regulating agent (4).

2. Sheet-like structure according to Claim 1, **characterized in that** the fibers (3) are at least partially coated with moisture-regulating agent (4).

3. Sheet-like structure according to Claim 1 or 2, **characterized in that** the polymer matrix (5) is arranged on the fibers (3), between the fibers (3), on the moisture-regulating agent (4) and/or in the moisture-regulating agent (4).

4. Sheet-like structure according to any of the preceding claims, **characterized in that** the at least one active ingredient is selected such that it can be released in a controlled and/or successive manner.

5. Sheet-like structure according to any of the preceding claims, **characterized in that** the polymer matrix is selected such that the at least one active ingredient can be released by dissolution of the polymer matrix (5).

6. Sheet-like structure according to any of the preceding claims, **characterized in that** the main part (2) comprises at least one further layer.

7. Sheet-like structure according to any of the preceding claims, **characterized in that** the at least one active ingredient is selected such that it can at least partially penetrate human skin and/or is suitable for external application and can produce an optical, odorous and/or tactile effect on the skin or in the skin.

8. Sheet-like structure according to any of the preceding claims, **characterized in that** the at least one active ingredient is selected from the group consisting of: vegetable oils; proteins; vitamins; enzymes; lipids; fruit acids; herbal active ingredients; animal active ingredients; hyaluronic acids and mixtures thereof.

9. Sheet-like structure according to any of Claims 3 to 8, **characterized in that** the polymer matrix (5) is self-adhesive.

10. Sheet-like structure according to any of the preceding claims, **characterized in that** the moisture-regulating agent (4) in the dry state has a weight per unit area, measured according to DIN standard ISO 9073-1, of 0.001 to 30 g/m².

11. Sheet-like structure according to any of the preceding claims, **characterized in that** the sheet-like structure (1) has a two- or three-dimensional structure of a face, a neckline, a hand, an arm, a foot, a leg or of another body part and/or the structure adapts to these body parts.

12. Sheet-like structure according to any of the preceding claims, **characterized in that** the textile material is configured as a non-woven fabric or non-woven material.

13. Process for preparing a sheet-like structure according to any of the preceding claims, comprising the process steps below:
a) treating a main part (2) composed of a textile material comprising fibers with a mixture comprising a precursor of a moisture-regulating agent (4) in the form of a polymerizable monomer or oligomer, and also a wetting agent and an initiator,
b) polymerizing the monomer or oligomer to give a moisture-regulating agent (4) and
c) providing the main part (2) with at least one active ingredient and a polymer matrix such that the at least one active ingredient is embedded in the polymer matrix and/or
d) providing the main part (2) with at least one active ingredient embedded in a polymer matrix.

14. Process according to Claim 13, **characterized in that** a cross-linker is added to the mixture.

15. Process according to Claim 14, **characterized in that** a degree of cross-linking is set in the range of 0.1 to 10%.

16. Use of a sheet-like structure according to any of Claims 1 to 12 for cosmetic treatment.

## Revendications

1. Structure plate (1), comprenant un corps de base (2) en un matériau textile muni d'au moins un agent actif, le corps de base (2) comprenant des fibres (3) et un super-absorbant en tant qu'agent de régulation de l'humidité (4), ledit au moins un agent actif étant incorporé dans une matrice polymère (5), **caractérisée en ce que** les fibres (3) sont reliées au moins partiellement par accouplement de matière avec l'agent de régulation de l'humidité (4).

2. Structure plate selon la revendication 1, **caractérisée en ce que** les fibres (3) sont au moins partiellement enrobées par l'agent de régulation de l'humidité (4) .

3. Structure plate selon la revendication 1 ou 2, **caractérisée en ce que** la matrice polymère (5) est agencée sur les fibres (3), entre les fibres (3), sur l'agent de régulation de l'humidité (4) et/ou dans l'agent de régulation de l'humidité (4).

4. Structure plate selon l'une quelconque des revendications précédentes, **caractérisée en ce que** ledit au moins un agent actif est choisi de telle sorte qu'il puisse être libéré de manière contrôlée et/ou successive.

5. Structure plate selon l'une quelconque des revendications précédentes, **caractérisée en ce que** la matrice polymère est choisie de telle sorte que ledit au moins un agent actif puisse être libéré par dissolution de la matrice polymère (5).

6. Structure plate selon l'une quelconque des revendications précédentes, **caractérisée en ce que** le corps de base (2) contient au moins une couche supplémentaire.

7. Structure plate selon l'une quelconque des revendications précédentes, **caractérisée en ce que** ledit au moins un agent actif est choisi de telle sorte qu'il puisse pénétrer au moins partiellement dans la peau humaine et/ou qu'il soit approprié pour l'application extérieure, et puisse produire un effet optique, odorant et/ou tactile sur la peau ou dans la peau.

8. Structure plate selon l'une quelconque des revendications précédentes, **caractérisée en ce que** ledit au moins un agent actif est choisi dans le groupe constitué par : les huiles végétales, les protéines, les vitamines, les enzymes, les lipides, les acides de fruits, les agents actifs végétaux, les agents actifs animaux, les acides hyaluroniques et leurs mélanges.

9. Structure plate selon l'une quelconque des revendications 3 à 8, **caractérisée en ce que** la matrice polymère (5) est munie d'un traitement auto-adhésif.

10. Structure plate selon l'une quelconque des revendications précédentes, **caractérisée en ce que** l'agent de régulation de l'humidité (4) présente à l'état sec un poids superficiel, mesuré selon la norme DIN ISO 9073-1, de 0,001 à 30 g/m².

11. Structure plate selon l'une quelconque des revendications précédentes, **caractérisée en ce que** la structure plate (1) présente une structure bi- ou tridimensionnelle d'un visage, d'un décolleté, d'une main, d'un bras, d'un pied, d'une jambe ou d'une autre partie du corps et/ou la structure s'adapte à cette partie du corps.

12. Structure plate selon l'une quelconque des revendications précédentes, **caractérisée en ce que** le matériau textile est configuré sous la forme d'un non-tissé ou d'un matériau non-tissé.

13. Procédé de fabrication d'une structure plate selon l'une quelconque des revendications précédentes, comprenant les étapes de procédé suivantes :
a) le traitement d'un corps de base (2) en un matériau textile, qui comprend des fibres, avec un mélange contenant un précurseur d'un agent de régulation de l'humidité (4) sous la forme d'un monomère ou oligomère polymérisable, ainsi qu'un agent de mouillage et un initiateur,
b) la polymérisation du monomère ou de l'oligomère pour former un agent de régulation de l'humidité (4), et
c) l'équipement du corps de base (2) avec au moins un agent actif et une matrice polymère, de telle sorte que ledit au moins un agent actif soit incorporé dans la matrice polymère et/ou
d) l'équipement du corps de base (2) avec au moins un agent actif incorporé dans une matrice polymère.

14. Procédé selon la revendication 13, **caractérisé en ce qu'**un agent de réticulation est ajouté au mélange.

15. Procédé selon la revendication 14, **caractérisé en ce qu'**un degré de réticulation dans la plage allant de 0,1 à 10 % est ajusté.

16. Utilisation d'une structure plate selon l'une quelconque des revendications 1 à 12 pour le traitement cosmétique.
